**Europäisches Patentamt**

**European Patent Office**

**Office européen des brevets**

(19)

(11) Veröffentlichungsnummer: **0 327 509 B1**

# EUROPÄISCHE PATENTSCHRIFT

(12)

(45) Veröffentlichungstag der Patentschrift:
22.05.91 Patentblatt 91/21

(51) Int. Cl.$^5$: **A61F 2/46**

(21) Anmeldenummer: **89810097.9**

(22) Anmeldetag: **03.02.89**

(54) **Chirurgisches Instrument zum Einsetzen von Hüftpfannen-Prothesen.**

(30) Priorität: **04.02.88 CH 386/88**

(43) Veröffentlichungstag der Anmeldung:
**09.08.89 Patentblatt 89/32**

(45) Bekanntmachung des Hinweises auf die
Patenterteilung:
**22.05.91 Patentblatt 91/21**

(84) Benannte Vertragsstaaten:
**CH DE ES FR GB IT LI**

(56) Entgegenhaltungen:
**EP-A- 0 121 002**
**EP-A- 0 140 642**
**WO-A-86/05384**

(56) Entgegenhaltungen:
**FR-A- 1 505 998**
**US-A- 4 305 394**
**US-A- 4 475 549**
**US-A- 4 611 587**
**US-A- 4 716 894**

(73) Patentinhaber: **Protek AG**
**Stadtbachstrasse 64**
**CH-3012 Bern (CH)**

(72) Erfinder: **Müller, Maurice E.**
**Melchenbühlweg 9**
**CH-3006 Bern (CH)**

(74) Vertreter: **Fischer, Franz Josef et al**
**c/o Bovard AG Optingenstrasse 16**
**CH-3000 Bern 25 (CH)**

EP 0 327 509 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft ein chirurgisches Instrument zum Richten und Einsetzen von Hüftpfannen-Prothesen bzw. von Metallschalen, die zur Aufnahme von Kunststoffpfannen dienen sowie einen Prüfansatz zur Ortung von unebenheiten und einen Aufsatz zum Einsetzen von Kunststoffpfannen. Mit dem Instrument wird eine kraftschlüssige Verbindung der einzusetzenden Prothesen erzielt. Weiter wird dem Chirurgen eine präzise, axiale Drehung von nicht rotationssymmetrischen Pfannen oder Metallschalen ermöglicht. Das Instrument ist so ausgebildet, dass es den Zugang zu wenigstens zwei vorhandenen Bohrungen zulässt, damit die Prothese mit Knochenschrauben in der korrekten Lage fixiert werden kann. Ein chirurgisches Instrument gemäß dem Oberbegriff des Anspruchs 1 ist aud der WO-A-86 05 384 bekannt.

Bei dem Ersatz des natürlichen Hüftgelenks durch eine Totalprothese wird nach dem heutigen Stand der Orthopädischen Chirurgie und dem Stand der Endoprothesentechnik als Gegenlager für den künstlichen, kugelförmigen Femurkopf bevorzugt eine Kunststoffpfanne verwendet, die im wesentlichen die Form einer halben Hohlkugel hat. Eine solche künstliche Hüftpfanne entspricht zum Beispiel einer Hohlkugelhälfte mit Aussendurchmesser 50 mm und Innendurchmesser 28 mm.

Eine solche künstliche Hüftpfanne kann im Os coxae nach verschiedenen Verfahren und mit verschiedenen Hilfsmitteln befestigt werden. Sie kann z.B. mit Knochenzement in die durch Kugelfräser vorbereitete natürliche Hüftpfanne, das Acetabulum, einzementiert werden.

Eine häufig bevorzugte Ausführung einer Hüftpfannenprothese besteht aus einer Schale aus Metall, die ebenfalls im wesentlichen die Form einer Hohlkugel-Hälfte hat und in die eine genau passende Kunststoffpfanne eingebracht wird, nachdem die Metallschale, im folgenden auch Aussenschale genannt, im Acetabulum befestigt worden ist.

Eine weitere Ausführung besteht aus einer äusseren Metallschale, mit der die innere Kunststoffpfanne bereits während der Herstellung unlösbar verbunden worden ist. Solche Schalen-/Pfanneneinheiten werden durch Schrauben, Verklemmen oder Knochenzement im Acetabulum befestigt.

Eine weitere Ausführung besteht aus einer Metallschale, auch als Pfannenschale oder Pfannendachschale bezeichnet, deren Innenform nicht mit der Aussenform der Kunststoffpfanne übereinstimmt und in die die Kunststoffpfanne mit Knochenzement einzementiert wird, nachdem die Metallschale mit Hilfe von Schrauben im Os coxae verankert worden ist.

Es gibt auch Kunststoff-Hüftpfannen, die nicht vollständig rotationssymmetrisch sind, sondern die auf einem Teil des Aequators eine Randüberhöhung zur Vermeidung von Femurkopfluxationen aufweisen. Metallschalen können zur besseren Befestigung und Kraftüberleitung auf einem Teil des Aequators mit einem Kragen oder Stützrand versehen sein.

Bei allen Hüftpfannen steht der Chirurg vor der Aufgabe, die künstliche Pfanne und somit gegebenenfalls auch die zugehörige Aussenschale in der richtigen Position, d.h. in der richtigen Ausrichtung in der Frontal-, Sagittal- und Transversalebene im Acetabulum zu verankern, damit die vom Femurkopf ausgeübte Kraft über die künstliche Hüftpfanne und gegebenenfalls Aussenschale so auf das Ox coxae übertragen wird, dass einerseits eine Luxation des Femurkopfes und andererseits eine Lockerung der künstlichen Pfanne so weit wie möglich verhindert werden.

Die Hüftpfannenprothese oder Aussenschale kann dank ihrer kugeligen Aussenfläche in dem mit Hilfe von Kugelfräsern vorbereiteten Knochenlager in verschiedene Positionen gebracht werden, d.h. die Winkel, unter denen die durch das Kugelzentrum und den Pol der Pfannenhalbkugel verlaufende Kugelachse die Sagittalebene und die Transversalebene des Patienten schneidet, können um einige Grade variiert werden. Ausserdem kann die künstliche Hüftpfanne oder Aussenschale um die eigene Achse gedreht werden. Ein Inklinationswinkel, das ist der Winkel zwischen der Kugelachse und der Sagittalebene, von 40 bis 45° und ein Anteversionswinkel, das ist der Winkel zwischen der Kugelachse und der Frontalebene, von 10 bis 15° haben sich bewährt.

Um Kunststoffpfannen annähernd unter diesen Winkeln einzementieren zu können, ist der von Maurice E. Müller entwickelte Pfanneneinschläger mit Zielgerät bekannt, der folgendermassen konstruiert ist : am vorderen Ende einer Metallstange mit Griff ist eine metallene Halbkugel aufgesetzt, die in die als Gegenlager für den künstlichen Femurkopf bestimmte Hohlkugel der künstlichen Hüftpfanne passt. Auf einer auf den Griff aufgeschobenen Hülse ist ein Kontrollzeiger so angebracht, dass er mit der genannten Metallstange einen Winkel von 45° bildet. Hält man das Instrument so, dass dieser Kontrollzeiger zur Longitudinalachse des Patienten parallel ist und sorgt man dafür, dass die durch das Kugelzentrum und den Pol der halbkugelförmigen Hüftpfanne verlaufende virtuelle Kugelachse mit der Metallstange des Instrumentes übereinstimmt, dann ist die gewünschte Inklination von 45° erreicht. Auf derselben Hülse ist ein zweiter Kontrollzeiger befestigt. Er befindet sich in der Ebene, die durch die Metallstange des Instruments verläuft und senkrecht auf der durch den ersten Kontrollzeiger und die Metallstange definierten Ebene steht.

Dieser zweite Kontrollzeiger bildet mit der distalen Verlängerung der Metallstange, also der Griffachse, einen Winkel von 78°. Hält man das Instrument so, dass sich dieser zweite Kontrollzeiger im Lot befindet, also senkrecht auf der Frontalebene steht, dann befindet sich die Hüftpfanne in der gewünschten Anteversion von 12°. Voraussetzung ist auch hier, dass die obengenannte virtuelle Kugelachse mit der Achse der Metallstange übereinstimmt.

Die exakte Uebereinstimmung der Instrumentenachse mit der durch das Kugelzentrum und den Pol der künstlichen Hüftpfanne und gegebenenfalls Aussenschale verlaufenden Achse ist mit Hilfe des bekannten Pfanneneinschlägers mit Zielgerät schwer erreichbar, da dieses Instrument nicht kraftschlüssig mit der Hüftpfannenprothese verbunden ist. Mit dem beschriebenen Pfanneneinschläger mit Zielgerät kann kein Drehmoment auf die zu positionierende Hüftpfanne ausgeübt werden. Dieses bisher verwendete Instrument ist daher nicht geeignet für das Positionieren von Pfannen mit Randüberhöhung oder Stützrand auf einem Teil des Aequators, d.h. für nicht rotationssymmetrische Pfannen oder von Metallschalen, die mit Schrauben im Os coxae befestigt werden und bei denen die für diese Schrauben vorgesehenen Bohrungen durch Verdrehen und Kippen der Pfanne oder Aussenschale in die richtige Position gebracht werden müssen.

Beim Einsetzen von Aussenschalen mit Knochenschrauben müssen die Löcher im Knochen vorgebohrt werden, wobei die Aussenschale als Bohrlehre dient. Das Setzinstrument muss also den Zugang zu wenigstens zwei Bohrungen erlauben, so dass die Löcher in den Knochen gebohrt werden können, dass gegebenenfalls ein Gewinde in den Knochen geschnitten werden kann und dass anschliessend wenigstens zwei Schrauben eingedreht werden können, wodurch die Position der Aussenschale dann festgelegt ist. Hieraus wird deutlich, dass das Setzinstrument zü jeder Zeit die Kontrolle der Position erlauben muss, dass wenigstens zwei Schrauben eindrehbar sein müssen, ohne das Setzinstrument zu entfernen und dass das Setzinstrument nach dem Eindrehen von wenigstens zwei Schrauben von der Pfanne leicht lösbar sein muss. Die Bedingungen der kraftschlüssigen Verbindung und leichten Lösbarkeit und Fixierbarkeit der Aussenschale mittels Knochenschrauben wird auch von einem bekannten Instrument, dem "Harris Cup Positioner" nicht erfüllt, der in der Druckschrift "The Total System" 85-037-9026-0326/REV, © 1984, Zimmer, Inc., abgebildet ist.

Es ist Aufgabe der vorliegenden Erfindung, ein chirurgisches Instrument zur Verfügung zu stellen, welches die obengenannten Voraussetzungen erfüllt und frei von den erwähnten Nachteilen der bekannten Instrumente ist.

Gegenstand der vorliegenden Erfindung ist das im Patentanspruch 1 definierte chirurgische Instrument sowie der Prüfansatz gemäß Anspruch 5 und der Aufsatz gemäß Anspruch 6.

Im folgenden wird die Erfindung und ihre Anwendung beispielsweise durch die Zeichnungen näher erläutert. Es zeigt:

Fig. 1 eine Oberansicht des Operationstisches, wobei die Lage des Patienten und des erfindungsgemässen chirurgischen Instrumentes ersichtlich ist,

Fig. 2 eine Seitenansicht des Operationstisches gemäss Fig. 1,

Fig. 3 eine perspektivische Darstellung des erfindungsgemässen Richt- und Setzinstrumentes,

Fig. 4 einen Aufriss im Längsschnitt gemäss IV-IV der Fig. 5 des in Fig. 3 dargestellten Instrumentes,

Fig. 5 eine Vorderansicht der Adapterseite des Richtund Setzinstrumentes gemäss Fig. 4,

Fig. 6 die Vorderansicht des Prüfaufsatzes,

Fig. 7 eine perspektivische Darstellung des Prüfaufsatzes,

Fig. 8 einen Längschnitt gemäss VIII-VIII der Fig. 6 durch den Prüfaufsatz gemäss den Fig. 6 und 7,

Fig. 9 das Zusammenspiel des Adapterstückes mit einem Spezialaufsatz zum Einsetzen von Kunststoffpfannen sowie eine passende Kunststoffpfanne im Längschnitt und `

Fig. 10 den in Fig. 9 dargestellten Aufsatz und das Adapterstück in perspektivischer Darstellung.

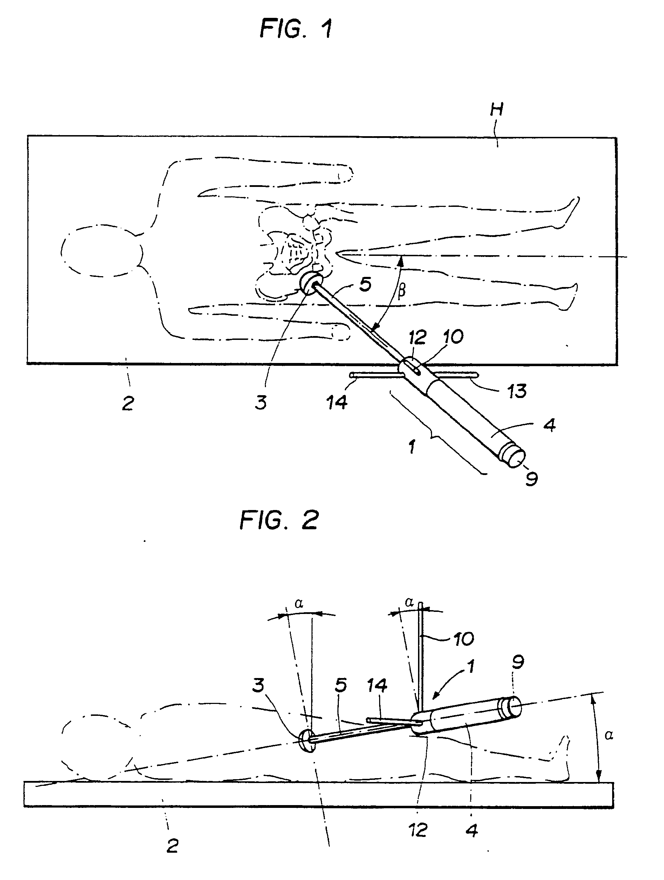

Zum besseren Verständnis soll anhand der Fig. 1 und 2 die Aufgabe der Positionierung einer Hüftpfannenprothese erläutert werden.

Der Patient liegt auf dem Operationstisch, der Ebene H. Die Ebene H ist parallel zu den Frontalebenen des Patienten. Die Longitudinalachse des Patienten ist parallel zur Ebene H und zur Längskante 2 des Operationstisches. Zum Zeitpunkt der Positionierung der künstlichen Hüftpfanne im Os coxae wird die Ebene H horizontal gestellt.

Die zu implantierende Hüftpfannenprothese 3 hat im wesentlichen die Form einer Hohlkugel. Es besteht nun die Aufgabe, die Hüftpfanne in der mittels eines Kugelfräsers vorbereiteten Mulde des Os coxae nach gegebenen Vorschriften zu positionieren. Diese Regeln sind:

Regel 1: Die Hüftpfanne soll mit dem Inklinationswinkel $\beta$, z.B. $\beta$ = 40°, implantiert werden. Das heisst: die Projektion der durch das Zentrum der Hüftpfannenhalbkugel und den Pol derselben gelegten virtuellen Kugelachse auf die Ebene H schneidet die Longitudinalachse des Patienten und somit auch die Längskante 2 des Operationstisches unter dem Winkel $\beta$.

Regel 2: Die Hüftpfanne soll mit einem Anteversionswinkel $\alpha$, z.B. $\alpha$ = 12°, implantiert werden. Die Verlängerung der in Regel 1 definierten virtuellen Kugelachse muss also die Ebene H unter dem Winkel $\alpha$ schneiden

Regel 3: Die künstliche Hüftpfanne muss so um ihre eigene Achse, die in Regel 1 definierte Kugelachse gedreht werden, dass sich der Stützrand oder/und die Randüberhöhung oder/und ihre für die Befestigung durch Schrauben vorgesehenen Bohrungen in der zur Vermeidung von Femurkopf-Luxationen bzw. zur Langzeitstabilität optimalen Position befinden.

Die oben beschriebenen Nachteile des bestehenden Pfanneneinschlägers mit Zielgerät werden durch das erfindungsgemässe Richt- und Setzinstrument 1 für Hüftpfannenprothesen beseitigt. Die Implantation gemäss den oben wiedergegebenen Regeln 1 bis 3 wird zeitsparend und mit grosser Genauigkeit ermöglicht. Dies wird vor allem durch eine starre, rotationssichere, aber nach dem Befestigen der Pfanne im Knochen einfach und durch Massnahmen ausserhalb des eigentlichen Operationsfeldes lösbare Verbindung zwischen Instrument und Pfanne erreicht.

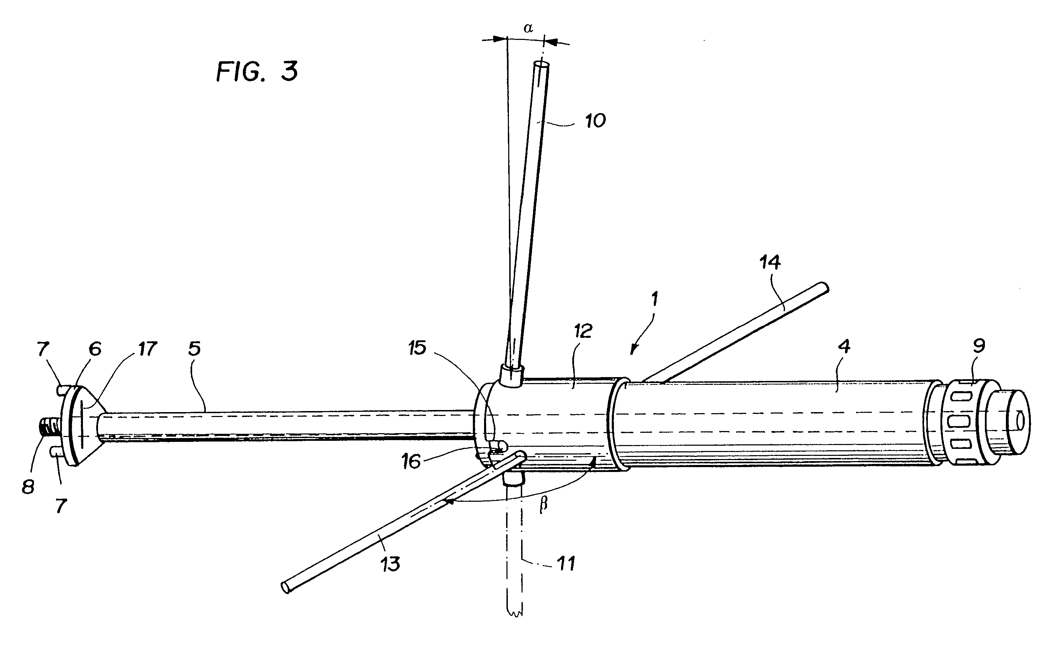

In Fig. 3 ist eine bevorzugte Ausführungsform des vollständigen erfindungsgemässen Richt- und Setzinstruments für eine Metallschale zur Aufnahme einer Kunststoffpfanne, im folgenden Aussenschale genannt, dargestellt.

Der Handgriff 4 ist fest mit dem Setzrohr 5 verbunden und das Adapterstück 6 mit den Passstiften 7 ist fest mit dem Setzrohr 5 verbunden. Beim Aufsetzen der Aussenschale auf das Adapterstück greifen die Passstifte 7 in Bohrungen der Aussenschale ein und stellen eine rotationssichere, eindeutige Verbindung zwischen der Aussenschale und dem Instrument her. Durch das Setzrohr 5 und den Handgriff 4 wird die Gewindestange 8 geführt, die durch Drehen des Knaufs 9 in ein passendes Gewinde im Pol der Aussenschale eingeschraubt wird.

Bei nicht rotationssymmetrischen Aussenschalen wird die Aussenschale mit Hilfe der Passstifte 7 in der Weise mit dem Richt- und Setzinstrument verbunden, dass sich die Aussenschale in der gewünschten Rotationslage befindet, wenn der Kontrollzeiger 10 bzw. 11 senkrecht auf der Ebene H steht. Um das Auffinden dieser Rotationslage zu erleichtern, ist auf dem Adapterstück 6 eine Markierungslinie 17 in der durch die Setzrohrachse und die Kontrollzeiger 10 und 11 definierten Ebene angebracht.

Zur richtigen Positionierung der Aussenschale unter dem Anteversionswinkel $\alpha$ dienen die Kontrollzeiger $A_R$, 10, für die Operation der rechten Hüfte, $A_L$, 11, für die Operation der linken Hüfte. Diese Zeiger sind durch Gewinde mit der Hülse 12 verbunden, so dass der jeweils nicht benötigte Zeiger herausgeschraubt werden kann. Die Achsen der Kontrollzeiger $A_R$, 10 und $A_L$, 11, bilden, wie Fig. 3 zeigt, mit dem Setzrohr den Winkel 90° + $\alpha$. Zur richtigen Positionierung der Hüftpfanne unter dem Inklinationswinkel $\beta$ dienen die Kontrollzeiger $B_R$, 13 und $B_L$, 14, die mit der Hülse 12 fest verbunden sind. Sie bilden, wie Fig. 1 zeigt, mit dem Setzrohr 5 den Winkel 180° − $\beta$.

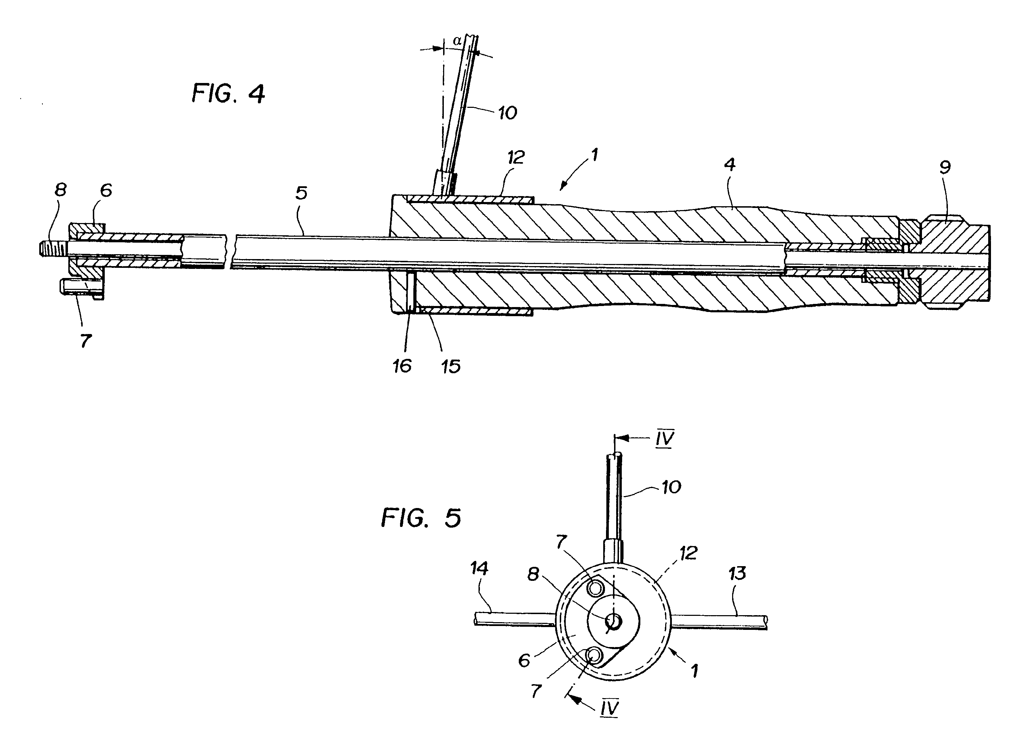

Durch den Schlitz 15 in der Hülse 12 und den Stift 16 im Handgriff ist die Hülse gegen Verdrehung gesichert. Fig. 4 zeigt einen Schnitt durch das in Fig. 3 perspektivisch dargestellte Setz- und Richtinstrument mit dem Handgriff 4, dem Setzrohr 5, dem fest mit dem Setzrohr verbundenen Adapterstück 6 mit Passstiften 7, der Gewindestange 8 mit Knauf 9 und Stift 16 zur rotationssicheren und eindeutigen Fixierung der auf den Handgriff aufschiebbaren Hülse 12 mit Kontrollzeigern 10, 11, 13, 14.

Fig. 5 zeigt die Vorderansicht des Adapterstückes 6 mit den Passstiften 7 und dem Gewindestück 8. Ebenfalls ersichtlich ist die Schnittebene IV-IV für Fig. 4.

Die Anwendung des erfindungsgemässen Instruments soll anhand der Fig. 1, 2 und 3 beschrieben werden. Die Fig. 1 und 2 zeigen die Lage des erfindungsgemässen Instrumentes 1 bezüglich des Patienten und des Operationstisches. Die Aussenschale aus Metall 23 der Hüftgelenkprothese 3 ist mit dem Richt- und Setzinstrument mittels der Gewindestange 8 und durch das Einführen von Passstiften 7 des Adapterstücks 6 rotations- und kippsicher verschraubt, so dass eine eindeutige Zuordnung der Aussenschale und der Kontrollzeiger 10, 11, 13, 14 sichergestellt ist.

Um die Hüftpfanne 1 unter dem Anteversionswinkel zu implantieren, muss die durch das Kugelzentrum und den Pol der Pfannenhalbkugel und der Aussenschale laufende Kugelachse um den Winkel $\alpha$ gegen die Frontalebene geneigt werden. Dasselbe gilt dann für das mit der Aussenschale fest verschraubte Setzrohr. Diese Lage wird, wie Fig. 2 zeigt, dadurch erreicht, dass das Instrument soweit geneigt wird, dass der Kontrollzeiger $A_R$, 10 bzw. $A_L$, 11, lotrecht steht. Die lotrechte Stellung kann vom Chirurgen oder einem Assistenten mit ausreichender Genauigkeit abgeschätzt werden.

Um die Hüftpfanne unter dem Inklinationswinkel zu implantieren, wird, wie in Fig. 1 ersichtlich, das Instrument so gehalten, dass der Zeiger $B_R$, 13 bzw. $B_L$, 14, zur Längskante 2 des Operationstisches parallel sind.

Die metallene Aussenschale kann jetzt mit zwei Schrauben im Knochen verschraubt werden, während sie unverändert mit dem Setz- und Richtinstrument verbunden bleibt. Erst nach dem Eindrehen von wenigstens zwei Schrauben in den Knochen wird das Setz- und Richtinstrument durch Drehen des Knaufs 9 der Gewindestange 8 von der Hüftpfanne gelöst. Jetzt können die weiteren Schrauben eingeschraubt werden. Nachdem die metallene Aussenschale durch die vorgesehene Anzahl von Schrauben im Acetabulum fixiert ist, kann die zugehörige Kunststoffpfanne in die Aussenschale eingeklemmt oder eingeschraubt werden.

Nach dem Einschrauben einer Metallschale in das Acetabulum mit Knochenschrauben und vor dem Einsetzen einer zugehörigen Kunststoffpfanne in die Metallschale ohne die Verwendung von Knochenzement muss sichergestellt sein, dass kein Schraubenkopf und keine etwa in Aussparungen der Metallschale eingebrachten Knochenstücke über die innere Hemisphäre herausragen, damit die Kunststoffpfanne einwandfrei in die Metallschale eingepresst werden kann. Für diese Ueberprüfung wurden zu jeder Grösse der Metallschale passende Prüfaufsätze geschaffen, die ebenfalls auf das Adapterstück des erfindungsgemässen Setz und Richtinstruments geschraubt werden können. Der Prüfaufsatz hat im wesentlichen die Form der in die Metallschale einzusetzenden Kunststoffpfanne, d.h. die Form einer Halbkugel, von der jedoch ein Segment derart abgeschnitten ist, dass die Schnittfläche in einem Winkel von ungefähr 15° zur Halbkugelachse verläuft.

Der Prüfaufsatz wird in die Metallschale eingeführt, wobei ein aussen am Pol des Prüfaufsatzes befindlicher Zapfen 21 in das Durchgangsloch mit Innengewinde im Pol der Metallschale eingreift, das zuvor zum Anschrauben der Metallschale an dem Richt- und Setzinstrument gedient hatte. Um diesen Zapfen als Achse wird nun der auf das Instrument geschraubte Prüfaufsatz gedreht. Steht der Kopf einer Knochenschraube, mit welcher die Metallschale in das Acetabulum geschraubt wurde, nach innen hervor, bleibt der Prüfaufsatz an der Grenzlinie der erwähnten Schnittfläche mit der Kugelfläche des Prüfaufsatzes hängen. Die Schraube muss dann weiter eingedreht oder entfernt werden.

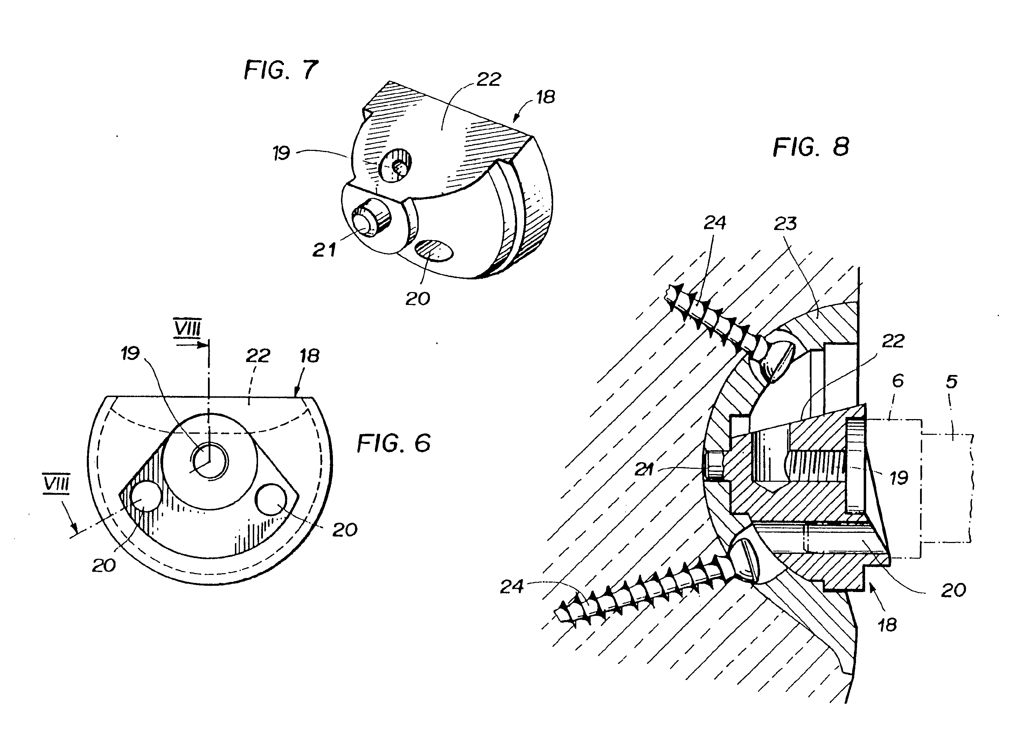

Eine bevorzugte Ausführungsform des Prüfaufsatzes ist in den Fig. 6 bis 8 dargestellt. Beim Aufsetzen des Prüfaufsatzes 18 auf das Adapterstück 6 des Richt- und Setzinstruments greifen die Passstifte 7 in die Bohrungen 20 ein und die Gewindestange 8 wird in die Bohrung mit Innengewinde 19 eingeschraubt. Dadurch, dass der Prüfaufsatz an der Schnittfläche 22 endet, ist der Einblick auf die Innenfläche der Metallschale 23 während der Prüfprozedur gewährleistet, so dass der die Rotation des Prüfaufsatzes um die durch den Zapfen 21 bestimmte Achse behindernde Schraubenkopf 24 geortet werden kann.

In Fig. 6 ist eine Projektion des Prüfaufsatzes aus der Richtung, aus welcher das Adapterstück eingesetzt wird, dargestellt. Es sind die Bohrungen 19 und 20 ersichtlich, wobei die Bohrung 19 mit Innengewinde zum Einschrauben der Gewindestange 8 dient und die Bohrungen 20 zum Eingreifen der Passstifte 7 dienen.

Fig. 7 zeigt den Prüfaufsatz in perspektivischer Darstellung, wobei die Schnittfläche gut zur Geltung kommt.

Fig. 8 zeigt einen Schnitt durch den Prüfaufsatz durch die Ebene VIII-VIII in Fig. 6. In der Darstellung ist ebenfalls die Metallschale 23 und das Adapterstück 6 sichtbar. Dargestellt ist auch eine Knochenschraube 24. Steht eine Schraube 24 im Innern der Oberfläche der Metallschale 23 vor, wird der Prüfaufsatz in seiner freien Drehbarkeit gehindert, d.h. die Kante der Schnittfläche 22 stösst an die hervorstehende Schraube 24. Die hervorstehende Schraube wird dann weiter eingedreht und der Prüfvorgang wiederholt. Ist der Prüfaufsatz frei drehbar, kann der Kunststoffeinsatz eingesetzt werden.

Für das Positionieren künstlicher Hüftpfannen aus Kunststoff, die mit Knochenzement in das Os coxae oder in eine Aussenschale einzementiert werden, wird das Richt- und Setzinstrument durch einen aufschraubbaren Aufsatz ergänzt, der es erlaubt, eine lösbare Verbindung zwischen dem Instrument und der Kunststoffpfanne herzustellen und die zum Positionieren und Einschlagen bzw. Eindrücken in den Knochenzement erforderlichen Kräfte auf die Kunststoffpfanne auszuüben.

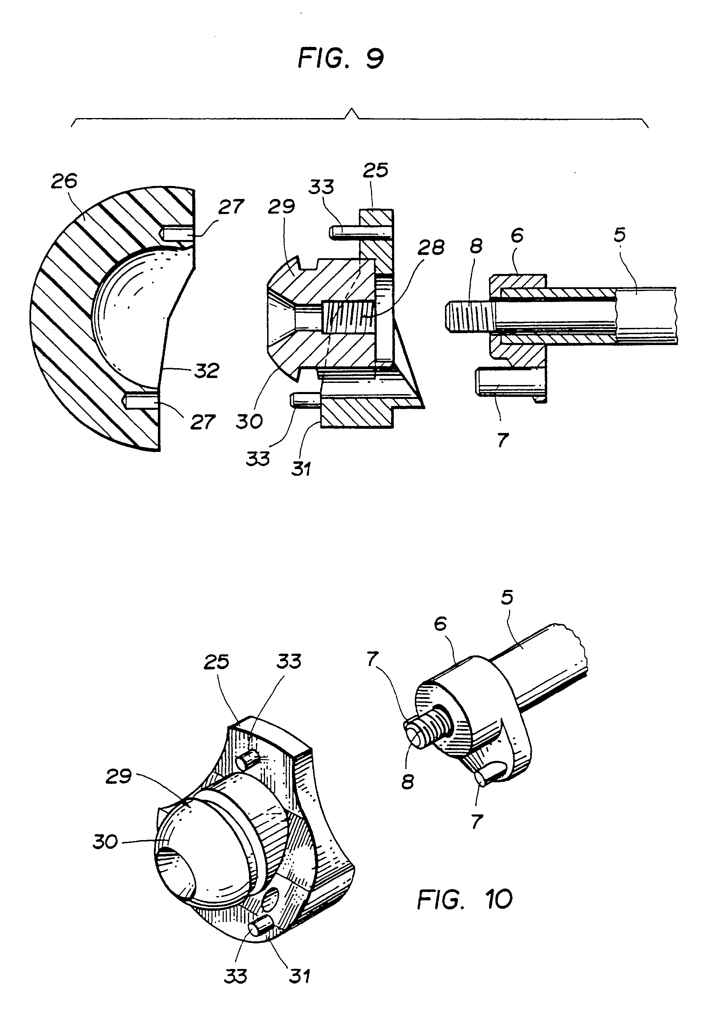

Fig. 9 zeigt den vorderen Teil des Setzrohres 5 und der Gewindestange 8 mit dem Adapterstück 6, ferner den Aufsatz 25 und eine Kunststoffpfanne 26. Dieser Aufsatz hat eine Kupplungsseite, in deren Bohrungen 27 das auf dem Setzrohr befestigte Adapterstück 6 eingreift und ein Durchgangsloch mit Innengewinde 28, in welches die Gewindestange des Richt- und Setzinstrumentes eingeschraubt werden kann. Auf der anderen Seite trägt der Aufsatz einen Einsatz 29 mit kugeligen Stirnflächen 30, der in die zu implantierende Kunststoffpfanne 26 passt. Der Einsatz 29 hat einen Kragen 31, dessen Form dem Rand 32 der Kunststoffpfanne angepasst ist. Die bevorzugt mit einem Abstand von 120° über den Kragen des Aufsatzes verteilten 3 Stifte 33 passen in die 3 Bohrungen 27 der Kunststoffpfanne 26. Die Durchmesser der Bohrungen 27 und der Stifte 33 sind so dimensioniert, dass sich die Kunststoffpfanne auf dem Aufsatz verklemmt.

Soll das erfindungsgemässe Setz- und Richtinstrument zum Positionieren und Einsetzen einer Kunststoffpfanne 26 verwendet werden, schraubt man den zu der Kunststoffpfanne passenden Aufsatz 25 mit Hilfe der Gewindestange 8 auf das Adapterstück 6 auf. Dann wird die Kunststoffpfanne auf den Aufsatz aufgeschoben, so dass die bevorzugt drei Stifte 33 in die entsprechenden Bohrungen 27 der Pfanne eingreifen. Die Pfanne sitzt dann hinreichend fest auf dem Richt- und Setzinstrument und kann, wie oben beschrieben, in die richtige Position gebracht werden. Beim Einschlagen oder Eindrücken der Pfanne in das mit Knochenzement versehene Acetabulum bzw. die Pfannendachschale 23 wird die Kraft über den Kragen 31 des Aufsatzes auf den Rand 32 der Pfanne übertragen, so dass die Gleitfläche der Pfanne nicht beschädigt wird. Nach Fixierung der Kunststoffpfanne 26, z.B. nach dem Aushärten des Knochenzementes kann das Instrument in Richtung der Achse des Setzrohres aus der Pfanne herausgezogen werden.

Fig. 10 zeigt den Aufsatz 25 für Kunststoffpfannen sowie das Adapterstück in perspektivischer Darstellung. Zum Einsetzen von Kunststoffpfannen in bereits im Acetabulum befestigte Metallschalen wie auch zum direkten Einzementieren in das Os coxae kann das gleiche Instrument verwendet werden.

Die durch den modularen Aufbau des Instruments ermöglichte Anwendung zum Positionieren von Metallschalen und Kunststoffpfannen mit und ohne Knochenzementbefestigung macht das Instrument preisgünstig und erleichtert die Lagerhaltung und Sterilisation im Krankenhaus.

**Ansprüche**

1. Chirurgisches Instrument zum Richten und Einsetzen von künstlichen Hüftpfannen und/oder Pfannenschalen zur Aufnahme solcher künstlicher Hüftpfannen, die im wesentlichen die Form einer halben Hohlkugel aufweisen, in einer durch die Winkel der Achse der Pfanne zu den Frontal-Transversal- und Sagittalebenen des Patienten festgelegten Position, welches Instrument im wesentlichen die folgenden Komponenten aufweist :
Setzrohr (5) mit Adapterstück (6) zur eindeutig bestimmten Befestigung der künstlichen Hüftpfanne oder Pfannenschale am Setzrohr und mit einer frei drehbaren Gewindestange (8), dadurch gekennzeichnet, daß am Setzrohr (5) Kontrollzeiger (10, 11, 13, 14) befestigt sind, die in zwei aufeinander senkrechten, die Achse des Setzrohres enthaltenden Ebenen liegen und die bestimmte, der vorgeschriebenen Anterversion und Inklination der Pfanne entsprechende Winkel α und β mit der Achse des Setzrohres bilden, dass die Gewindestange (8) durch das Setzrohr (5) und as Adapterstück (6) verläuft, wobei das Gewinde im Bereich des Adapterstückes hervortritt, damit das Adapterstück (6) und die Gewindestange (8) mit der Künstlichen Hüftanne oder Pfannenschale auf ihrer Innenseite im Polbereich eine kraftschulüssige, lösbare Verbindung bilden können, und daß das Adapterstück (6) und die Gewindestange (8) derart ausgebildet sind, dass vorhandene Bohrungen auf den Breitengraden 0 bis 40° der Pfannenschalen-Halbkugel für das Einschrauben von Knochenschrauben zugänglich sind, damit mindestens eine provisorische Schraubbefestigung in der korrekten Lage im Os coxae ermöglicht wird.

2. Instrument nach Anspruch 1, dadurch gekennzeichnet, dass die Gewindestange (8) an der dem Adapterstück entgegengesetzten Seite einen Drehknauf (9) aufweist, welcher zum Eindrehen oder Lösen der Gewindestange in einer im Polbereich mit einer Bohrung mit Innengewinde versehenen künstlichen Hüftpfanne bzw. Hüftpfannenschale dient, damit die zur Lösung der Verbindung notwendigen Handgriffe in einiger Entfernung von der Operationswunde vorgenommen werden können und die Sicht auf die Operationswunde jederzeit gewährleistet ist.

3. Instrument nach Anspruch 1 oder 2, dadurch gekennzeichnet, dass das Setzrohr (5) mit einem Handgriff (4) versehen ist, an welchem eine Hülse (12) mit den angebrachten Kontrollzeigern (10, 11, 13, 14) abnehmbar befestigt ist, damit die Hülse (12) mit den Kontrollzeigern zur präzisen Festlegung der räumlichen Lagen von verschiedenen rechten oder linken Hüftpfannen bzw. Pfannenschalen am Os coxae ausgewechselt werden kann.

4. Instrument gemäss Anspruch 3, dadurch gekennzeichnet, dass die Hülse (12) mit den Kontrollzeigern (10, 11, 13, 14) auf den Griff (4) aufschiebbar ist und dass die Position der Hülse durch einen Schlitz (15) und einen Stift (16) am Griff (4) des Instrumentes festgelegt wird.

5. Prüfaufsatz (18) zur Ortung von Unebenheiten, insbesondere hervorstehende Köpfe von Knochenschrauben in eingesetzten Hüftpfannenschalen (23), dadurch gekennzeichnet, dass der Prüfaufsatz (18) eine im wesentlichen in die Hüftpfannenschale (23) passende halbkugelförmige Form aufweist, wobei im Polgebiet ein hervorstehender Zapfen (21) vorhanden ist, der in das Durchgangsloch mit Innengewinde der Hüftpfannenschale (23) passt, wobei jedoch ein Segment dieses Prüfaufsatzes derart abgeschnitten ist, dass eine Schnittfläche (22) in einem Winkel von etwa 15° zur Halbkugelachse vorhanden ist und dass der Prüfaufsatz eine Bohrung (19) mit Innengewinde in der Kugelachse auf der dem Pol abgewandten Seite für die Gewindestange (8) und Bohrungen (20) für Passstifte (7) des Adapterstückes (6) des chirurgischen Instrumentes gemäss Anspruch 1 aufweist.

6. Aufsatz (25) zum Einsetzen von Kunststoffpfannen von küstlichen Hüftpfannenprothesen, dadurch gekennzeichnet, dass der Aufsatz ein Durchgangsloch mit Innengewinde (28) aufweist, in welches die Gewindestange (8) des chirurgischen Instruments gemäss Anspruch 1 einschraubbar ist, dass der Aufsatz (25) auf der dem Adapterstück entgegengesetzten Seite eine solche Form aufweist, dass er in die Hüftpfanne passt, und daß seitlich Stifte (33) vorhanden sind, die in Bohrungen (27) passen, die auf der Aussenseite der Hüftpfanne vorhanden sind.

**Claims**

1. A surgical instrument for positioning and insertion of artificial hip cups and/or shells for receiving such artificial hip cups having substantially the shape of a hollow hemisphere, in a position determined by the angle of the axis of the cup to the frontal, transverse and sagittal planes of the patient, which instrument has essentially the following components :
A setting tube (5) with adapter piece (6) for the exactly determined attachment of the artificial hip cup or shell to the setting tube and with a freely rotatable threaded rod (8), characterized in that alignment bars (10, 11, 13, 14) are affixed to the setting tube (5) lying in two planes perpendicular to one another and containing the axis of the setting tube and forming with the axis of the setting tube predetermined angles α and β corresponding to the prescribed anteversion and inclination of the cup,

in that the threaded rod (8) runs through the setting tube (5) and the adapter piece (6), the thread protruding in the vicinity of the adapter piece so that the adapter piece (6) and the threaded rod (8) can form a frictional, detachable connection with the artificial hip cup or cup shell on the inside thereof in the polar region, and in that the adapter piece (6) and the threaded rod (8) are dimensioned in such a way that bores present on the latitudes 0 to 40° of the cup-shell hemisphere are accessible for driving in bone screws so that at least a temporary screw connection in the correct position in the Os coxae is made possible.

2. The instrument of claim 1, characterized in that the threaded rod (8) has on the side opposite the adapter piece a rotary knob (9) serving to screw in or loosen the threaded rod in an artificial hip cup or acetabular shell provided in its pole region with a bore having an internal thread, so that the manipulations necessary for releasing the connection can be carried out at some distance from the surgical wound and a clear view of the surgical wound is ensured at all times.

3. The instrument of claim 1 or 2, characterized in that the setting tube (5) is provided with a handle (4) to which a sleeve (12) with the alignment bars (10, 11, 13, 14) affixed is detachably fastened so that the sleeve (12) with the alignment bars can be interchanged for precise determination of the spatial positions of different right or left hip cups or cup shells on the Os coxae.

4. The instrument of claim 3, characterized in that the sleeve (12) with the alignment bars (10, 11, 13, 14) is slidable onto the handle (4) and in that the position of the sleeve is determined by a slot (15) and a pin (16) on the handle (4) of the instrument.

5. An insert checking guide (18) for locating irregularities, particularly protruding heads of bone screws in inserted acetabular shells (23), characterized in that the insert checking guide (18) has a substantially hemispheric shape fitting into the acetabular shell (23), there being in the polar region a projecting stud (21) which fits into the continuous hole with internal thread of the acetabular shell (23), a segment of the insert checking guide being cut off in such a way that there is a cut surface (22) at an angle of about 15° to the hemisphere axis, and the insert checking guide has a bore (19) with internal thread for the threaded rod (8) in the sphere axis on the side remote from its pole, and bores (20) for the adjustment pegs (7) of the adapter piece (6) of the surgical instrument of claim 1.

6. A top piece (25) for inserting plastic cups of artificial acetabular prostheses, characterized in that the top piece has a continuous hole with an internal thread (28) into which the threaded rod (8) of the surgical instrument of claim 1 can be screwed, in that the top piece (25) has on the side remote from the adapter piece a shape such that it fits into the hip cup, and in that there are lateral pins (33) fitting in bores (27) existing on the rim of the hip cup.

## Revendications

1. Instrument chirurgical pour aligner etplacer des cavités d'os iliaques synthétiques et/ou des supports pour maintenir de telles cavités d'os iliaques, qui présentent essentiellement la forme d'une demi-sphère creuse selon une position déterminée par l'angle de l'axe de la cavité par rapport au plan frontal, transversal et sagittal des patients, ledit instrument comprenant essentiellement les éléments suivants : un tube de mise en place (5) avec une pièce d'adaptation (6) pour une fixation précise des cavités d'os iliaques synthétiques ou des supports au tube de mise en place et avec tige filetée (8) pivotant librement, caractérisé en ce que des aiguilles indicatrices (10, 11, 13, 14) sont fixées au tube de mise en place, contenues dans deux plans perpendiculaires entre eux et contenant l'axe du tube de mise en place et qui représentent précisément les angles α et β avec l'axe du tube correspondant à l'angle de sens opposé et d'inclinaison de la cavité, et en ce que la tige filetée (8) passe à travers le tube de mise en place (5) et la pièce d'adaptation (6), le filetage ressortant dans la région de la pièce d'adaptation, afin que la pièce d'adaptation (6) et la tige filetée (8) puissent constituer une liaison détachable maintenue avec force, avec la cavité d'os iliaque synthétique ou le support, sur leur côté intérieur, dans la région polaire, et en ce que la tige filetée (8) est ainsi formée, que des alésages sont prévus sur les degrés 0 à 40° de la demi-sphère de la cavité de l'os iliaque pour visser des vis à os, afin qu'au moins une fixation provisoire à vis soit possible selon la position correcte dans l'os coxae.

2. Instrument selon la revendication 1, caractérisé en ce que la tige filetée (8) présente un bouchon pivotant (9) sur le côté opposé à la pièce d'adaptation, lequel sert à visser ou dévisser la tige filetée dans l'un des alésages avec un filetage intérieur, prévus dans la région polaire de la cavité de l'os iliaque synthétique, respectivement du support, afin que pour démonter la liaison, une poignée nécessaire puisse être prévue à une certaine distance de l'ouverture d'opération, afin que la vue sur l'ouverture d'opération soit tout le temps garantie.

3. Instrument selon l'une des revendications 1 ou 2, caractérisé en ce que le tube de mise en place (5) est pourvu d'une poignée (4) sur laquelle une douille (12) amovible est fixée, comportant les aiguilles indicatrices (10, 11, 13, 14), afin que la douille (12) avec les aiguilles indicatrices puissent être inversées pour une fixation précise des situations spatiales de différentes cavités d'os iliaques, respectivement supports gauche ou droite à l'os coxae.

4. Instrument selon la revendication 3, caractérisé en ce que la douille (12) avec les aiguilles indicatrices (10, 11, 13, 14) est déplaçable et que la position de la douille est déterminée par une rainure (15) et une pointe (16) sur la poignée (4) de l'instrument.

5. Garniture d'essai (18) pour le positionnement de bosses, en particulier des têtes saillantes de vis à os dans un support de cavité d'os iliaque (23), caractérisé en ce que la garniture d'essai (18) comprend une forme en demi-sphère s'adaptant substantiellement dans la cavité de l'os iliaque (23), une cheville saillante (21) étant prévue dans la région polaire, qui passe à travers le trou de passage comprenant un filetage intérieur de la cavité de l'os iliaque (23), un segment de cette garniture d'essai étant toutefois coupé de la même manière, en ce qu'une surface coupée (22) selon un angle d'environ 15° par rapport à l'axe de la demi-sphère est prévue, et en ce que la garniture d'essai comprend un alésage (19) avec un filetage intérieur, selon l'axe de la sphère sur le côté polaire détourné, pour la tige filetée (8) et des alésages (20) pour des pointes de positionnement (7) de la pièce d'adaptation (6) de l'instrument chirurgical selon la revendication 1.

6. Garniture (25) pour l'insertion de cavités synthétiques de prothèses d'os iliaques synthétiques, caractérisé en ce que la garniture comprend un alésage traversant avec un filetage intérieur (28) dans lequel la tige filetée (8) de l'instrument chirurgical selon la revendication 1 peut être vissée, et en ce que la garniture (25) comprend une telle forme, sur un côté opposé à la pièce d'adaptation, qu'il passe dans la cavité de l'os iliaque, et en ce que des pointes latérales (33) sont prévues, qui passent dans les alésages (27) prévus sur les faces extérieures de la cavité de l'os iliaque.

## FIG. 1

## FIG. 2

FIG. 3

FIG. 4

FIG. 5

FIG. 8

FIG. 7

FIG. 6

## FIG. 9

## FIG. 10